# EUROPEAN PATENT APPLICATION

(11) **EP 3 884 959 A1**
(43) Date of publication of application: **29.09.2021**
(21) Application number: 19886626.1
(22) Date of filing: 19.11.2019
(51) Int. Cl.: A61K 39/395, A61P 3/00, A61P 43/00

(54) **INHIBITOR OF CELLULAR IRON UPTAKE**

(30) Priority: 20.11.2018 JP 2018217548; 13.09.2019 JP 2019167013
(71) Applicant: Perseus Proteomics Inc., Tokyo 153-0041 (JP)
(72) Inventor: ZHANG Lilin, Tokyo 153-0041 (JP); NOMURA Fumiko, Tokyo 153-0041 (JP); KATSUMI Keiko, Tokyo 153-0041 (JP); KOTAKA Romi, Tokyo 153-0041 (JP); OHIRA Yuta, Tokyo 153-0041 (JP)
(74) Representative: Hoffmann Eitle
(86) International application number: PCT/JP2019/045227
(87) International publication number: WO 2020/105621

(57) **Abstract**

It is an object of the present invention to provide an agent for inhibiting iron uptake into cells wherein the agent targets TfR, and an agent for inhibiting the binding between human Tf and human TfR. The present invention provides an agent for inhibiting iron uptake into cells which comprises an antibody which recognizes the amino acids at positions 629 to 633 of a human transferrin receptor.

SEQUENCE LISTING
<110>  Perseus Proteomics Inc.
<120>  Agent for inhibition of incorporation of iron in cells
<130>  191983A
<160>  10
<170>  PatentIn version 3.5
<210>  1
<211>  5
<212>  PRT
<213>  human
<400>  1
Ser Tyr Gly Met His
1               5
<210>  2
<211>  17
<212>  PRT
<213>  human
<400>  2
Val Ile Ser Tyr Asp Gly Ser Asn Lys Tyr Tyr Ala Asp Ser Val Lys
1               5                   10                  15
Gly

<210>  3
<211>  14
<212>  PRT
<213>  human
400>  3
Asp Ser Asn Phe Trp Ser Gly Tyr Tyr Ser Pro Val Asp Val
1               5                   10
<210>  4
<211>  13
<212>  PRT
<213>  human
<400>  4

Thr Arg Ser Ser Gly Ser Ile Ala Ser Asn Ser Val Gln
1               5                   10
<210>  5
<211>  8
<212>  PRT
<213>  human
<400>  5
Tyr Glu Asp Thr Gln Arg Pro Ser
1               5
<210>  6
<211>  10
<212>  PRT
<213>  human
<400>  6
Gln Ser Tyr Asp Ser Ala Tyr His Trp Val
1               5                   10
<210>  7
<211>  123
<212>  PRT
<213>  human
<400>  7
Asp Val Gln Leu Val Gln Ser Gly Gly Gly Val Val Gln Pro Gly Arg
1               5                   10                  15
Ser Leu Arg Leu Ser Cys Ala Ala Ser Gly Phe Pro Phe Lys Ser Tyr
            20                  25                  30
Gly Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val

50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Gly Glu Asp Thr Ala Val Tyr Tyr Cys
                85                  90                  95
Ala Arg Asp Ser Asn Phe Trp Ser Gly Tyr Tyr Ser Pro Val Asp Val
            100                 105                 110
Trp Gly Gln Gly Thr Thr Val Thr Val Ser Ser
        115                 120
<210>  8
<211>  111
<212>  PRT
<213>  human
<400>  8
Asn Phe Met Leu Thr Gln Pro His Ser Val Ser Glu Ser Pro Gly Lys
1               5                   10                  15
Thr Val Thr Ile Ser Cys Thr Arg Ser Ser Gly Ser Ile Ala Ser Asn
            20                  25                  30
Ser Val Gln Trp Tyr Gln Gln Arg Pro Gly Ser Ala Pro Ile Thr Val
        35                  40                  45
Ile Tyr Glu Asp Thr Gln Arg Pro Ser Gly Val Pro Asp Arg Phe Ser
    50                  55                  60
Gly Ser Ile Asp Ser Ser Ser Asn Ser Ala Ser Leu Thr Ile Ser Gly
65                  70                  75                  80
Leu Gln Thr Glu Asp Glu Ala Asp Tyr Tyr Cys Gln Ser Tyr Asp Ser
                85                  90                  95
Ala Tyr His Trp Val Phe Gly Gly Gly Thr Lys Leu Ala Val Leu
            100                 105                 110
<210>  9
<211>  760
<212>  PRT
<213>  human
<400>  9
Met Met Asp Gln Ala Arg Ser Ala Phe Ser Asn Leu Phe Gly Gly Glu
1               5                   10                  15
Pro Leu Ser Tyr Thr Arg Phe Ser Leu Ala Arg Gln Val Asp Gly Asp
            20                  25                  30
Asn Ser His Val Glu Met Lys Leu Ala Val Asp Glu Glu Glu Asn Ala
        35                  40                  45
Asp Asn Asn Thr Lys Ala Asn Val Thr Lys Pro Lys Arg Cys Ser Gly
    50                  55                  60
Ser Ile Cys Tyr Gly Thr Ile Ala Val Ile Val Phe Phe Leu Ile Gly
65                  70                  75                  80
Phe Met Ile Gly Tyr Leu Gly Tyr Cys Lys Gly Val Glu Pro Lys Thr
                85                  90                  95
Glu Cys Glu Arg Leu Ala Gly Thr Glu Ser Pro Val Arg Glu Glu Pro
            100                 105                 110
Gly Glu Asp Phe Pro Ala Ala Arg Arg Leu Tyr Trp Asp Asp Leu Lys
        115                 120                 125
Arg Lys Leu Ser Glu Lys Leu Asp Ser Thr Asp Phe Thr Gly Thr Ile
    130                 135                 140
Lys Leu Leu Asn Glu Asn Ser Tyr Val Pro Arg Glu Ala Gly Ser Gln
145                 150                 155                 160
Lys Asp Glu Asn Leu Ala Leu Tyr Val Glu Asn Gln Phe Arg Glu Phe
                165                 170                 175
Lys Leu Ser Lys Val Trp Arg Asp Gln His Phe Val Lys Ile Gln Val
            180                 185                 190
Lys Asp Ser Ala Gln Asn Ser Val Ile Ile Val Asp Lys Asn Gly Arg
        195                 200                 205
Leu Val Tyr Leu Val Glu Asn Pro Gly Gly Tyr Val Ala Tyr Ser Lys
    210                 215                 220

Ala Ala Thr Val Thr Gly Lys Leu Val His Ala Asn Phe Gly Thr Lys
225                 230                 235                 240
Lys Asp Phe Glu Asp Leu Tyr Thr Pro Val Asn Gly Ser Ile Val Ile
                245                 250                 255
Val Arg Ala Gly Lys Ile Thr Phe Ala Glu Lys Val Ala Asn Ala Glu
            260                 265                 270
Ser Leu Asn Ala Ile Gly Val Leu Ile Tyr Met Asp Gln Thr Lys Phe
        275                 280                 285
Pro Ile Val Asn Ala Glu Leu Ser Phe Phe Gly His Ala His Leu Gly
    290                 295                 300
Thr Gly Asp Pro Tyr Thr Pro Gly Phe Pro Ser Phe Asn His Thr Gln
305                 310                 315                 320
Phe Pro Pro Ser Arg Ser Ser Gly Leu Pro Asn Ile Pro Val Gln Thr
                325                 330                 335
Ile Ser Arg Ala Ala Ala Glu Lys Leu Phe Gly Asn Met Glu Gly Asp
            340                 345                 350
Cys Pro Ser Asp Trp Lys Thr Asp Ser Thr Cys Arg Met Val Thr Ser
        355                 360                 365
Glu Ser Lys Asn Val Lys Leu Thr Val Ser Asn Val Leu Lys Glu Ile
    370                 375                 380
Lys Ile Leu Asn Ile Phe Gly Val Ile Lys Gly Phe Val Glu Pro Asp
385                 390                 395                 400
His Tyr Val Val Val Gly Ala Gln Arg Asp Ala Trp Gly Pro Gly Ala
                405                 410                 415
Ala Lys Ser Gly Val Gly Thr Ala Leu Leu Leu Lys Leu Ala Gln Met
            420                 425                 430
Phe Ser Asp Met Val Leu Lys Asp Gly Phe Gln Pro Ser Arg Ser Ile
        435                 440                 445
Ile Phe Ala Ser Trp Ser Ala Gly Asp Phe Gly Ser Val Gly Ala Thr
    450                 455                 460
Glu Trp Leu Glu Gly Tyr Leu Ser Ser Leu His Leu Lys Ala Phe Thr
465                 470                 475                 480
Tyr Ile Asn Leu Asp Lys Ala Val Leu Gly Thr Ser Asn Phe Lys Val
                485                 490                 495
Ser Ala Ser Pro Leu Leu Tyr Thr Leu Ile Glu Lys Thr Met Gln Asn
            500                 505                 510
Val Lys His Pro Val Thr Gly Gln Phe Leu Tyr Gln Asp Ser Asn Trp
        515                 520                 525
Ala Ser Lys Val Glu Lys Leu Thr Leu Asp Asn Ala Ala Phe Pro Phe
    530                 535                 540
Leu Ala Tyr Ser Gly Ile Pro Ala Val Ser Phe Cys Phe Cys Glu Asp
545                 550                 555                 560
Thr Asp Tyr Pro Tyr Leu Gly Thr Thr Met Asp Thr Tyr Lys Glu Leu
                565                 570                 575
Ile Glu Arg Ile Pro Glu Leu Asn Lys Val Ala Arg Ala Ala Ala Glu
            580                 585                 590
Val Ala Gly Gln Phe Val Ile Lys Leu Thr His Asp Val Glu Leu Asn
        595                 600                 605
Leu Asp Tyr Glu Arg Tyr Asn Ser Gln Leu Leu Ser Phe Val Arg Asp
    610                 615                 620
Leu Asn Gln Tyr Arg Ala Asp Ile Lys Glu Met Gly Leu Ser Leu Gln
625                 630                 635                 640
Trp Leu Tyr Ser Ala Arg Gly Asp Phe Phe Arg Ala Thr Ser Arg Leu
                645                 650                 655
Thr Thr Asp Phe Gly Asn Ala Glu Lys Thr Asp Arg Phe Val Met Lys
            660                 665                 670
Lys Leu Asn Asp Arg Val Met Arg Val Glu Tyr His Phe Leu Ser Pro
        675                 680                 685
Tyr Val Ser Pro Lys Glu Ser Pro Phe Arg His Val Phe Trp Gly Ser
    690                 695                 700
Gly Ser His Thr Leu Pro Ala Leu Leu Glu Asn Leu Lys Leu Arg Lys
705                 710                 715                 720
Gln Asn Asn Gly Ala Phe Asn Glu Thr Leu Phe Arg Asn Gln Leu Ala

                725                 730                 735
Leu Ala Thr Trp Thr Ile Gln Gly Ala Ala Asn Ala Leu Ser Gly Asp
            740                 745                 750
Val Trp Asp Ile Asp Asn Glu Phe
        755                 760
<210> 10
<211> 28
<212> PRT
<213> Artificial Sequence
<220>
<223> Description of Artificial Sequence: Recombinant tag
<400> 10
Ala Ala Ala Arg Gly Gly Pro Glu Gln Lys Leu Ile Ser Glu Glu Asp
1               5                   10                  15
Leu Asn Ser Ala Val Asp His His His His His His
            20                  25

## Description

### Technical Field

The present invention relates to an agent for inhibiting iron uptake into cells, and an agent for inhibiting the binding between a human transferrin and a human transferrin receptor.

### Background Art

A transferrin receptor (TfR) has been identified to be a cell membrane structure for incorporating an iron which was bound to a transferrin (Tf) into cells, which is present on reticulocytes (Non-Patent Document 1). It has been known that TfR is expressed in placental trophoblast cells, activated lymphocytes, tumor cells, and the like.

Patent Document 1 describes that phage antibodies (scFv antibodies) reacting with TfR present on cancer cells were obtained according to human antibody phage display library, and such scFv antibodies were then converted to IgGs, so as to produce complete human IgG antibodies. Patent Document 1 also describes that at least one amino acid was modified in the variable region CDR of the obtained complete human anti-TfR antibody to produce an anti-TfR antibody suitable for clinical application.

### Prior Art Documents

### Patent Documents

Patent Document 1: International Publication WO2014/073641

### Non-Patent Documents

Non-Patent Document 1: J Clin Invest 1963; 42, 314-326
Non-Patent Document 2: Gene. 1991 Dec 15; 108(2): 193-9.

### Summary of Invention

### Object to be Solved by the Invention

It is an object of the present invention to provide an agent for inhibiting iron uptake into cells wherein the agent targets TfR and an agent for inhibiting the binding between human Tf and human TfR.

### Means for Solving the Object

As a result of intensive studies directed towards achieving the aforementioned object, the present inventors have found that an antibody which recognizes an amino acid sequence at a predetermined position in human TfR can inhibit the binding between human Tf and human TfR, and can further inhibit iron uptake into cells, thereby completing the present invention.

Specifically, according to the present invention, the following invention is provided.
(1) An agent for inhibiting iron uptake into cells, which comprises an antibody which recognizes the amino acids at positions 629 to 633 of a human transferrin receptor.
(2) The agent for inhibition according to (1), which inhibits the binding between a human transferrin and a human transferrin receptor, so as to inhibit iron uptake into cells.
(3) The agent for inhibition according to (1) or (2), wherein the antibody has a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.
(4) The agent for inhibition according to any one of (1) to (3), wherein the antibody has a heavy chain as set forth in SEQ ID NO: 7 and a light chain as set forth in SEQ ID NO: 8.
(5) The agent for inhibition according to any one of (1) to (4), wherein the antibody is a human antibody or a humanized antibody.
(6) The agent for inhibition according to any one of (1) to (5), wherein the antibody is an antibody fragment selected from the group consisting of Fab, Fab', F(ab')₂, a single-chain antibody (scFv), a dimerized V region (Diabody), a disulfide stabilized V region (dsFv), and a peptide comprising CDR.
(7) The agent for inhibition according to any one of (1) to (6), which is used for the treatment of a disease or a symptom associated with excessive iron uptake into cells.
(8) An agent for inhibition the binding between a human transferrin and a human transferrin receptor, which comprises an antibody which recognizes the amino acids at positions 629 to 633 of a human transferrin receptor.
(9) The agent for inhibition according to (8), wherein the antibody has a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.
(10) The agent for inhibition according to (8) or (9), wherein the antibody has a heavy chain as set forth in SEQ ID NO: 7 and a light chain as set forth in SEQ ID NO: 8.
(11) The agent for inhibition according to any one of (8) to (10), wherein the antibody is a human antibody or a humanized antibody.
(12) The agent for inhibition according to any one of (8) to (11), wherein the antibody is an antibody fragment selected from the group consisting of Fab, Fab', F(ab')₂, a single-chain antibody (scFv), a dimerized V region (Diabody), a disulfide stabilized V region (dsFv), and a peptide comprising CDR.
(13) The agent for inhibition according to any one of (8) to (12), which is used for the treatment of a disease or a symptom associated with excessive iron uptake into cells.

(A) There is provided a method of inhibiting iron uptake into cells, which comprises administering an antibody which recognizes the amino acids at positions 629 to 633 of a human transferrin receptor to a subject.
(B) There is provided a method of inhibiting the binding between a human transferrin and a human transferrin receptor, which comprises administering an antibody which recognizes the amino acids at positions 629 to 633 of a human transferrin receptor to a subject.
(C) A method of treating a disease or a symptom associated with excessive iron uptake into cells, wherein the method comprises administering an antibody which recognizes the amino acids at positions 629 to 633 of a human transferrin receptor to a subject.
(D) An antibody which recognizes the amino acids at positions 629 to 633 of a human transferrin receptor, for use in inhibition of iron uptake into cells.
(E) An antibody which recognizes the amino acids at positions 629 to 633 of a human transferrin receptor, for use in inhibition of the binding between a human transferrin and a human transferrin receptor.
(F) An antibody which recognizes the amino acids at positions 629 to 633 of a human transferrin receptor, for use in the treatment of a disease or a symptom associated with excessive iron uptake into cells.
(G) Use of an antibody which recognizes the amino acids at positions 629 to 633 of a human transferrin receptor, for the production of an agent for inhibiting iron uptake into cells.
(H) Use of an antibody which recognizes the amino acids at positions 629 to 633 of a human transferrin receptor, for the production of an agent for inhibiting the binding between a human transferrin and a human transferrin receptor.
(I) Use of an antibody which recognizes the amino acids at positions 629 to 633 of a human transferrin receptor, for the production of an agent for treating a disease or a symptom associated with excessive iron uptake into cells.

### Advantageous Effects of Invention

According to the present invention, an agent for inhibiting iron uptake into cells and an agent for inhibiting the binding between human Tf and human TfR are provided. The agent for inhibition of the present invention are useful for the treatment of a disease or a symptom associated with excessive iron uptake into cells. The agent for inhibition of the present invention can be used to suppress the growth of cells having high iron requirements, such as erythroblasts.

### Brief Description of Drawings

[Fig.1] Fig. 1 shows the sites at which a point mutation is made on individual TfR mutant fragments.
[Fig.2] Fig. 2 shows the reactivity of TfR436 with soluble wild-type TfR (sTfR) and with TfR mutant fragments.
[Fig.3] Fig. 3 shows the Tf-TfR binding inhibitory activity of TfR436.
[Fig.4] Fig.4 shows the effects of ferric ammonium citrate on the cell growth suppressing effect of a TfR436 antibody.
[Fig.5] Fig. 5 shows a change in the amount of iron in a K562 cell line by addition of a TfR436 antibody.

### Embodiment of Carrying out the Invention

Hereinafter, the present invention will be described in more details.

### Definitions and General Techniques

Unless otherwise specified in the present description, scientific terms used regarding the present invention have meanings which are generally understood by a person skilled in the art. In general, nomenclatures and techniques applied to the cell and tissue culture, molecular biology, immunology, microbiology, genetics, protein and nucleic acid chemistry, and hybridization, which are described in the present description, are well known in the present technical field, and thus, are commonly used.

The methods and techniques of the present invention are carried out in accordance with conventional methods which are well known in the present technical field, in such ways as described in a variety of general reference documents cited and discussed throughout the present description and more specific reference documents, unless otherwise specified.

### TfR

Human transferrin receptor (TfR) is a single-pass transmembrane protein (SEQ ID NO: 9) comprising 760 amino acids, and it is encoded by human chromosome 3. This protein has also been known as a CD71 antigen, and it is considered that this protein is associated with incorporation of iron into cells and cell growth. The TfR of the present invention is not particularly limited in terms of structure. Thus, human TfR includes all of a monomer, a polymer, an intact form expressed on a cell membrane, a soluble form constituted in an extracellular region, a truncated form, a mutation form caused by genetic mutation, deletion, etc., and a form which has undergone posttranslational modification by phosphorylation or the like.

### React and Reactivity

The terms "react" and "reactivity" have the same meanings in the present description, unless otherwise specified. That is, these terms mean that an antibody recognizes an antigen. The antigen used herein may be any of an intact TfR expressed on a cell membrane, a truncated form, and a soluble form. In addition, the antigen may be either a TfR having a three-dimensional structure or a denatured TfR. Examples of a means for examining reactivity include flow cytometry (FACS), enzyme-linked immunosorbent assay (ELISA), Western blotting, microfluorescence measuring technique (FMAT), surface plasmon resonance (Biacore), immunostaining, and immunoprecipitation.

The antibody used in flow cytometry may be either an antibody labeled with a fluorescent substance such as FITC or with biotin, or an unlabeled antibody. A fluorescently-labeled avidin, a fluorescently-labeled anti-human immunoglobulin antibody, or the like is used, depending on the presence or absence of labeling of the antibody used and the type thereof. Reactivity can be evaluated by adding a sufficient amount of anti-TfR antibody (generally having a final concentration of 0.01 to 10 µg/mL) to an analyte, and then by comparing the obtained reactivity with the reactivity with a negative control antibody or a positive control antibody.

### Antibody

In the present description, the following abbreviations (in the parentheses) are used in accordance with the customs, as necessary.
Heavy chain (H chain), light chain (L chain), heavy chain variable region (VH), light chain variable region (VL), complementarity determining region (CDR), first complementarity determining region (CDR1), second complementarity determining region (CDR2), third complementarity determining region (CDR3), heavy chain first complementarity determining region (VH CDR1), heavy chain second complementarity determining region (VH CDR2), heavy chain third complementarity determining region (VH CDR3), light chain first complementarity determining region (VL CDR1), light chain second complementarity determining region (VL CDR2), and light chain third complementarity determining region (VL CDR3).

In the present description, the term "antibody" has the same definitions as immunoglobulin, and should be understood as generally known in the present technical field. Specifically, the term "antibody" is not limited by any given specific method for producing the antibody. For example, the term "antibody" includes, but is not limited to, a recombinant antibody, a monoclonal antibody, and a polyclonal antibody.

In the present description, the term "human antibody is used to mean any given antibody, in which the sequences of a variable region and a constant region are human sequences. This term includes antibodies which have human sequences and are modified, for example, to remove cysteine which may cause a possible decrease in immunogenicity, an increase in affinity, and undesirable folding. This term also includes antibodies produced in non-human cells by recombination, which enable glycosylation that is not specific to human cells. These antibodies can be prepared in various ways.

In the present description, the term "humanized antibody" means a non-human-derived antibody, in which amino acid residues characteristic for a non-human antibody sequence are substituted with residues found in positions corresponding to those of a human antibody. This "humanization" process is considered to reduce the immunogenicity of the obtained antibody in human. It would be understood that a non-human-derived antibody can be humanized using a technique well known in the present technical field. Please refer to, for example, Winter et al., Immunol. Today 14: 43-46 (1993). The target antibody can be produced by an engineering approach via a recombination DNA technique of substituting CH1, CH2, CH3, a hinge domain, and/or a framework domain with those of the corresponding human sequence. For example, WO92/02190, and U. S. Patent Nos. 5,530,101, 5,585,089, 5,693,761, 5,693,792, 5,714,350 and 5,777,085 can be referred. In the present description, the term "humanized antibody" includes a chimeric human antibody and a CDR-grafted antibody, within the definitions thereof.

The sequence of a framework region (FR) in a variable region of the antibody is not particularly limited, unless it substantially affects the specific binding ability of the antibody to the corresponding antigen. The FR region of a human antibody is preferably used, but it is also possible to use FR regions of animal species other than humans (e.g. a mouse, a rat, etc.).

In one aspect of the antibody, the antibody comprises a constant region as well as a variable region (e.g. IgG antibody). The sequence of such a constant region is not particularly limited. For example, the constant region of a known human antibody can be used. The heavy chain constant region (CH) of a human antibody is not particularly limited, as long as it belongs to a human immunoglobulin (hereinafter referred to as "hlgG"). Those of hIgG class are preferable, and any one of subclasses belonging to the hlgG class, such as hIgG1, hIgG2, hIgG3 or hIgG4, may be used. On the other hand, the light chain constant region (CL) is not particularly limited, as long as it belongs to hIg, and those of κ class or λ class can be used. In addition, constant regions of animal species other than humans (e.g. a mouse or a rat) can also be used.

In the present description, the term "modified form" or "modified antibody" is used to mean that the amino acid sequence of the variable region (CDR sequences and/or FR sequences) of a parent antibody comprises a substitution, deletion, addition and/or insertion of one or multiple amino acids.

In the present invention, the "parent antibody" means a TfR436 antibody which has a VH comprising the amino acid sequence shown in SEQ ID NO: 7 and a VL comprising the amino acid sequence shown in SEQ ID NO: 8. In the amino acid sequence, one or several (for example, 1 to 8, preferably 1 to 5, more preferably 1 to 3, and particularly preferably 1 or 2) amino acids are deleted, added, substituted and/or inserted. As a method of preparing the amino acid sequence of an antibody having a binding ability to TfR, which has been well known to a person skilled in the art, a method of introducing a mutation into a protein has been known. For instance, such a skilled person could prepare a modified antibody functionally equivalent to an antibody having a TfR-binding activity by appropriately introducing a mutation into the amino acid sequence of the antibody having a TfR-binding activity according to a site-directed mutagenesis (Hashimoto-Gotoh, T, Mizuno, T, Ogasahara, Y, and Nakagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci USA. 82, 488-492). Thus, an antibody comprising a mutation of one or several amino acids in the variable region or constant region thereof and having a binding activity to TfR can also be used.

In the present description, the phrase "an activity equivalent to the activity of the parent antibody" is used to mean that the human TfR-binding activity of a certain antibody is equivalent to that of the parent antibody thereof. The term "equivalent" does not necessarily mean the same level of activity. The activity may be increased, or the activity may also be decreased, as long as the antibody has the activity. An antibody having a decreased activity may be an antibody having an activity that is, for example, 30% or more, preferably 50% or more, more preferably 80% or more, further preferably 90% or more, and particularly preferably 95% or more of the activity of the original antibody.

The term "binding activity" means the activity of an antibody to recognize an antigen. This antigen may be an intact TfR expressed on a cell membrane, a truncated form, or a soluble form. In addition, the antigen may be either a TfR having a three-dimensional structure or a denatured TfR. Examples of a means for examining the binding activity include flow cytometry (FACS), enzyme-linked immunosorbent assay (ELISA), Western blotting, microfluorescence measuring technique (FMAT), and surface plasmon resonance (Biacore).

The Tf-TfR binding inhibitory activity of the antibody can be measured according to the method described in the after-mentioned "Example 2(2): Comparison between TfR436 antibody and the antibody developed by another company in terms of inhibition of Tf-TfR binding." A TfR solution is dispensed on a substrate (a 96-well plate, etc.) and is then left at rest for immobilization, and it is blocked. Subsequently, an HRP-labeled Tf solution is dispensed thereon, and the antibody is further added thereto, followed by performing a reaction at room temperature. Thereafter, the substrate is washed, and a coloring reagent (TMB, etc.) is then added thereto for a reaction. After that, the absorbance is measured using a plate reader. By performing the above-described operations, the Tf-TfR binding inhibitory activity of the antibody can be evaluated.

The inhibitory activity of the antibody on iron uptake into cells can be measured according to the method described in the after-mentioned "Example 3: Effects of ferric ammonium citrate on cell growth suppressing effect of TfR436 antibody." Specifically, cells are suspended in a culture medium, and the suspension is then seeded on a substrate (a 96-well plate, etc.). A solution is prepared by serially diluting the antibody to obtain suitable concentrations, and the prepared solution is then added to the above-described cells. Then, ferric ammonium citrate is further added thereto. The cells are cultured for a predetermined period of time, and then, individual wells are fully stirred. Thereafter, the cell culture solution is transferred into another plate (for example, a V-bottom 96-well plate, etc.). An aliquot of the solution is sucked using FACS Calibur (BD), and the number of events is then measured. The number which is four times the obtained number of events is defined to be the number of cells per well. The mean value of the number of cells in a well, to which neither the antibody nor ferric ammonium citrate is added, is set at 100%, and the growth rate in each treatment is calculated. When the antibody suppresses the growth of the cells in a concentration-dependent manner and the termination of the cell growth is released by addition of ferric ammonium citrate, it is demonstrated that the antibody inhibits iron uptake into cells.

The antibody is not limited by its origin, and it may be an antibody derived from any animal, such as a human antibody, a mouse antibody, or a rat antibody. Also, the present antibody may be a chimeric antibody or a humanized antibody. In a preferred aspect of the antibody, the antibody of the present invention is a human antibody.

The antibodies may be different from one another in terms of amino acid sequence, molecular weight, isoelectric point, the presence or absence of a sugar chain or the form thereof, etc., depending on the after-mentioned cells or hosts which produce the antibodies, or a purification method. For example, an antibody which undergoes a modification after it has been translated to the amino acid sequence described in the present description is also included in the present invention. Moreover, an antibody which has undergone a posttranslational modification on a site other than those for the known posttranslational modification is also included in the present invention. Furthermore, when the antibody is allowed to express in prokaryotic cells such as *Escherichia coli,* a methionine residue is added to the N-terminus of the amino acid sequence of the original antibody. In the present invention, such an antibody may also be used. An antibody which has undergone a posttranslational modification on a site other than those for the known posttranslational modification is also included in the present invention.

### Production of Antibody

### (1) scFv reacting with antigen using phage display library

The antibody can be prepared by several methods which are known in the present technical field. For example, using a phage display technique, a library comprising a repertoire of antibodies having various affinity for TfR can be provided. Subsequently, such a library can be screened to identify and isolate antibodies against TfR. Preferably, the phage library is a scFv phage display library which is generated using human VL and VH cDNA which has been prepared from mRNA isolated from human B cells. A method of preparing and screening such a library is known in the present technical field. A genetic substance is recovered from phage clones exhibiting reactivity which have been screened using a human TfR as an antigen. By analyzing the selected phage gene, the DNA sequences of VH and VL encoding the variable region of a human antibody binding to the antigen can be determined. Using this scFv sequence, IgG is prepared from scFv, so as to obtain a human antibody.

### (2) Preparation of IgG from scFv (preparation of human antibody)

An H chain or L chain expression vector is produced, and it is then allowed to express in a host cell. Thereafter, the secreted supernatant is recovered and is then purified, so as to obtain a human antibody. Alternatively, a human antibody can also be obtained by allowing VH and VL to express in a single vector (tandem type). These methods are well known, and can be carried out with reference to WO92/01047, WO92/20791, WO93/06213, WO93/11236, WO93/19172, WO95/01438, WO95/15388, WO97/10354, etc.

Specifically, DNA encoding VH is ligated to another DNA molecule encoding a heavy chain constant region (CH1, CH2 and CH3), so as to obtain a full-length heavy chain gene. The sequence of a human heavy chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The heavy chain constant region may be the constant region of IgG1, IgG2, IgG3, IgG4, IgA, IgE, IgM or IgD. The most preferred constant region is constant region of IgG1 or IgG2. The constant region sequence of IgG1 may include any given various alleles or allotypes known to be generated among different individuals, such as Gm (1), Gm (2), Gm (3) or Gm (17). These allotypes correspond to a substitution of amino acids naturally-occurring in the constant region of IgG1.

DNA encoding VL is ligated to another DNA molecule encoding the light chain constant region CL, so as to obtain a full-length L chain gene (and a Fab light chain gene). The sequence of a human light chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The light chain constant region may be the constant region of κ or λ. The κ constant region may include any given various alleles known to be generated among different individuals, such as Inv (1), Inv (2) or Inv (3). The λ constant region may be derived from any one of the three λ genes.

The thus obtained DNA encoding an H chain or L chain is inserted into a vector to produce an expression vector, and the produced expression vector is then allowed to express in a host cell. Thereafter, the secreted supernatant is recovered and purified to obtain a human antibody. Examples of the expression vector include a plasmid, retrovirus, adenovirus, adeno-associated virus (AAV), plant viruses such as cauliflower mosaic virus or tobacco mosaic virus, a cosmid, YAC, and EBV-derived episome. An expression vector and an expression regulatory sequence are selected, so that they are suitable for a host cell used for expression. An antibody light chain gene and an antibody heavy chain gene can be inserted into different vectors, or the two genes can also be inserted into a single expression vector. An antibody gene is inserted into an expression vector by a standard method (for example, ligation of a complementary restriction site on an antibody gene fragment to a vector, or blunt-ended ligation applied when no restriction sites are present).

A favorable vector encodes a functionally completed human CH or CL immunoglobulin sequence having a suitable restriction site, which has been produced by an engineering approach such that any given VH or VL sequence can be easily inserted and then expressed therein, as described above. In such a vector, splicing generally takes place between a splice donor site in the inserted J region and a splice acceptor site preceding a human C domain, or such splicing also takes place in a splice region existing in a human CH exon. Polyadenylation and transcription termination take place in a natural chromosomal site downstream of a coding region. A recombinant expression vector can also encode a signal peptide which promotes the secretion of an antibody chain derived from a host cell. An antibody chain gene can be cloned into a vector, such that a signal peptide can be ligated in-frame to the amino terminus of an immunoglobulin chain. The signal peptide may be either an immunoglobulin signal peptide or a heterogeneous signal peptide (namely, it may be a non-immunoglobulin protein-derived signal peptide).

An expression vector used for the antibody may also have sequences such as a sequence for regulating replication of the vector in a host cell (e.g. a replication origin) or a selective marker gene sequence, as well as an antibody gene and a regulatory sequence. The selective marker gene promotes selection of a host cell into which a vector has been introduced. For instance, the selective marker generally imparts resistance to drugs such as G418, hygromycin or methotrexate to a host cell into which the vector has been introduced. Preferred selective marker genes include a dihydrofolate reductase (DHFR) gene (used in selection/amplification of methotrexate as a dhfr-host cell), a neomycin phosphotransferase gene (used in G418 selection), and a glutamate synthase gene.

A host cell is transformed with an antibody gene expression vector produced by the above-described method. Any type of cell may be used as a host cell, as long as it can produce the antibody of the present invention. Examples of such a host cell include bacteria, yeast, animal cells, insect cells, and plant cells. Among these cells, animal cells are preferable. Examples of the animal cells include Chinese hamster ovary cells CHO/dhfr(-) and CHO/DG44, monkey-derived cells COS (A. Wright & S. L. Morrison, J. Immunol. 160, 3393-3402 (1998)), and SP2/O cells (mouse myeloma) (K. Motmans et al., Eur. J. Cancer Prev. 5, 512-5199 (1996), R. P. Junghans et al., Cancer Res. 50, 1495-1502 (1990)). For transformation, a lipofectin method (R. W. Malone et al., Proc. Natl. Acad. Sci. USA 86, 6007 (1989), P. L. Felgner et al., Proc. Natl. Acad. Sci. USA 84, 7413 (1987)), an electroporation method, a calcium phosphate method (F. L. Graham & A. J. van der Eb, Virology 52,456-467 (1973)), a DEAE-Dextran method, and the like are preferably applied.

A transformant is cultured, and a human antibody is then separated from the cells of the transformant or a culture medium thereof. For separation/purification of the antibody, methods such as centrifugation, ammonium sulfate fractionation, salting-out, ultrafiltration, affinity chromatography, ion exchange chromatography and gel filtration chromatography can be used by appropriately combining them.

### Antibody Fragments

An antibody fragment can be produced based on the antibody, or based on the sequence information of a gene encoding the antibody. Examples of the antibody fragment include Fab, Fab', F(ab')₂, scFv , and dsFv antibodies.

Fab is obtained by digesting IgG by papain in the presence of cysteine. It is an antibody fragment with a molecular weight of approximately 50,000, which is constituted with L chain and H chain variable regions, and an H chain fragment consisting of a CH1 domain and a portion of a hinge region. In the present invention, Fab can be obtained by digesting the above-described antibody by papain. In addition, Fab can also be prepared by incorporating DNA encoding a portion of the H chain and the L chain of the above-described antibody into a suitable vector, then performing transformation with the resulting vector, and then obtaining it from the transformant.

Fab' is an antibody fragment with a molecular weight of approximately 50,000, which is obtained by cleaving a disulfide bond between the H chains of the below-mentioned F(ab')₂. In the present invention, Fab' can be obtained by digesting the above-described antibody by pepsin, and then cleaving a disulfide bond with a reducing agent. In addition, as with Fab, Fab' can also be prepared by genetic engineering using DNA encoding the Fab'.

F(ab')₂ is an antibody fragment with a molecular weight of approximately 100,000, which is obtained by binding, via a disulfide bond, one fragment (Fab') constituted with L chain and H chain variable regions and an H chain fragment consisting of a CH1 domain and a portion of a hinge region, to the other fragment (Fab'), which is obtained by digesting IgG by pepsin. In the present invention, F(ab')₂ can be obtained by digesting the above-described antibody by pepsin. In addition, as with Fab, F(ab')₂ can also be prepared by genetic engineering using DNA encoding the F(ab')₂.

scFv is an antibody fragment obtained by ligating the C-terminus of one chain of Fv consisting of an H chain variable region and an L chain variable region to the N-terminus of the other chain thereof, using a suitable peptide linker, so as to form a single chain. (GGGGS)₃ having high flexibility can be used, for example, as such a peptide linker. For instance, DNA encoding the H chain variable region and L chain variable region of the above-described antibody and DNA encoding a peptide linker are used to construct DNA encoding a scFv antibody, and the thus constructed DNA is then incorporated into a suitable vector. Thereafter, scFv can be prepared from a transformant obtained by transformation with the a forementioned vector.

dsFv is a Fv fragment obtained by introducing a Cys residue into a suitable site in each of an H chain variable region and an L chain variable region, and then stabilizing the H chain variable region and the L chain variable region by a disulfide bond. The site in each chain, into which the Cys residue is to be introduced, can be determined based on a conformation predicted by molecular modeling. In the present invention, for example, a conformation is predicted from the amino acid sequences of the H chain variable region and L chain variable region of the above-described antibody, and DNA encoding each of the H chain variable region and the L chain variable region, into which a mutation has been introduced based on such prediction, is then constructed. The thus constructed DNA is incorporated into a suitable vector. Thereafter, dsFv can be then prepared from a transformant obtained by transformation with the aforementioned vector.

Further, it is also possible to ligate the scFv antibody, the dcFv antibody or the like using a suitable linker, or to fuse such an antibody fragment with streptavidin, so as to multimerize the antibody fragment.

### Pharmaceutical Composition and Preparation

A pharmaceutical composition and a preparation, both of which comprise the agent for inhibition of the present invention, are also included in the scope of the present invention.

The agent for inhibition of the present invention can be used for the treatment of a disease or a symptom associated with excessive iron uptake into cells.

The disease or the symptom associated with excessive iron uptake into cells may be, for example, iron overload.

The pharmaceutical composition and the preparation preferably comprise a physiologically acceptable diluent or carrier, as well as the antibody. The pharmaceutical composition and the preparation may also be a mixture with another drug. Examples of a suitable carrier used herein may include a normal saline, a phosphate buffered saline, a phosphate buffered saline with glucose, and a buffered saline, but the examples are not limited thereto. Otherwise, the antibody is freeze-dried, and when needed, the aforementioned buffered aqueous solution may be added thereto to reconstitute the antibody, and the thus reconstituted antibody may be then used. Examples of the dosage form of the preparation include: oral administration, which uses a tablet, a capsule, a granule, a powder agent, a syrup, etc.; and parenteral administration, which includes injections (subcutaneous injection, intravenous injection, intramuscular injection, intraperitoneal injection, etc.), percutaneous administration, transmucosal administration, transnasal administration, transpulmonary administration, the use of a suppository, etc. The preparation comprising the pharmaceutical composition of the present invention may be administered alone, or it may also be used in combination with other drugs.

The applied dose of the agent for inhibition of the present invention is different depending on symptom, age, body weight, etc. In general, in the case of oral administration, the agent for inhibition is administered at a dose of approximately 0.01 mg to 1,000 mg per day per adult, in terms of the amount of an antibody contained therein. Such a dose can be administered once or divided over several administrations per day. On the other hand, in the case of parenteral administration, the agent for inhibition can be administered at a dose of approximately 0.01 mg to 1,000 mg for a single administration via subcutaneous injection, intramuscular injection or intravenous administration.

The present invention will be described in more details in the following examples. However, these examples are not intended to limit the scope of the present invention.

### Examples

In the following Examples, the TfR436 antibody described in paragraphs 0090 and 0091 of International Publication WO2014/073641 was used.

The CDR sequences of the TfR436 antibody are shown below.
VH CDR1:SYGMH (SEQ ID NO: 1)
VH CDR2:VISYDGSNKYYADSVKG (SEQ ID NO: 2)
VH CDR3:DSNFWSGYYSPVDV (SEQ ID NO: 3)
VL CDR1:TRSSGSIASNSVQ (SEQ ID NO: 4)
VL CDR2:YEDTQRPS (SEQ ID NO: 5)
VL CDR3:QSYDSAYHWV (SEQ ID NO: 6)

The VH sequence and VL sequence of the TfR436 antibody are shown below. TfR436 VH (SEQ ID NO: 7)

DVQLVQSGGGVVQPGRSLRLSCAASGFPFKSYGMHWVRQAPGKGLEWVAVISYD
GSNKYYADSVKGRFTISRDNSKNTLYLQMNSLRGEDTAVYYCARDSNFWSGYYSP
VDVWGQGTTVTVSS

TfR436 VL (SEQ ID NO: 8)

NFMLTQPHSVSESPGKTVTISCTRSSGSIASNSVQWYQQRPGSAPITVIYEDTQRPS
GVPDRFSGSIDSSSNSASLTISGLQTEDEADYYCQSYDSAYHWVFGGGTKLAVL

### Example 1: Identification of binding site of TfR436 antibody

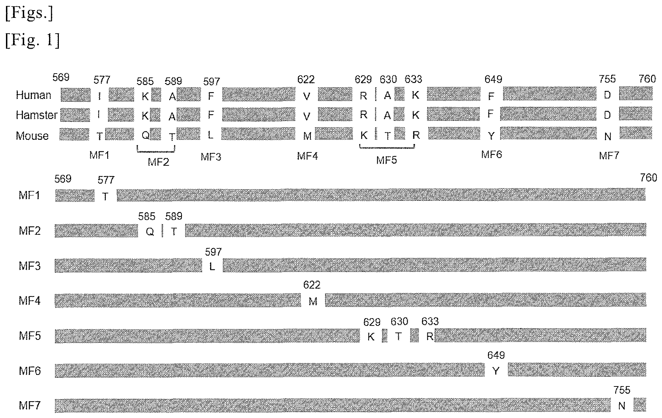

The TfR436 antibody did not cross-react with mouse TfR, but exhibited cross-reactivity with hamster TfR. The amino acid sequence of the transferrin (TF)-binding site (the amino acids at positions 569 to 760) in human TfR was aligned with the amino acid sequences of hamster TfR and mouse TfR. The amino acids of the human TfR sequence, which were identical to those of hamster TfR but were different from those of mouse TfR, were picked up. As shown in Fig. 1, the picked-up amino acids were subjected to point mutation, so as to produce soluble TfR mutant fragments.

### (1) Production of soluble wild-type TfR (sTfR) and TfR mutant fragments (MF1 to MF7)

A nucleotide sequence encoding a human TfR extracellular domain (the amino acids at positions 89 to 760) or individual TfR mutant fragments (MF1 to MF7) shown in Fig. 1, and AAARGGPEQKLISEEDLNSAVDIIIIIIIIIIII (SEQ ID NO: 10), were all synthesized. The synthesized genes were each inserted into the multicloning site of a vector prepared by incorporating a neomycin resistance gene and a DHFR gene into the expression vector pCAGGS (Non-Patent Document 2.: Niwa et al. 1991), so as to produce a pCAGGS-Neo-DHFR-sTFR-myc-his expression plasmid. Using Expifectamine (Invitrogen), the above-described plasmid was transfected into Expi293 cells (Invitrogen), and the obtained cells were then cultured at 37°C, in 8 % CO2, at 135 rpm for 5 days. Thereafter, a culture supernatant was recovered by centrifugation, and a HisTrapHP (GE Healthcare) column was then connected with AKTA prime (GE Healthcare). After that, 20 mM Imidazole/DPBS was used as a binding buffer, 500 mM Imidazole/DPBS was used as an elution buffer, and sTfR or each of MF1 to MF7 was purified. Using Zeba spin column (Thermo scientific), the eluted protein was subjected to buffer exchange with 30 mM HEPES, 5% trehalose, pH 7.2.

### (2) Identification of binding site of TfR436 antibody

The above-purified sTfR or each of MF1 to MF7 was diluted with PBST (Phosphate Buffered Saline with Tween20,TaKaRa) to prepare 7 steps of dilution series by 3-fold dilution from 600 ng/mL. Thereafter, the diluted solution was dispensed in an amount of 100 µL/well into a Ni-NTA HisSorb Strips 96-well plate (QIAGEN), and the plate was placed on a shaker and was then reacted at room temperature. One hour later, the plate was washed with PBST Buffer five times, and the TfR436 antibody (1 µg/mL) was dispensed in an amount of 100 µL/well into the plate, was then placed on a shaker, and was then reacted at room temperature for 1 hour. Thereafter, the plate was washed with PBST Buffer five times, and a 50,000-fold diluted secondary antibody, F(ab')₂ Fragment Anti-Human IgG Fcγ (Jackson Immuno Research) was then dispensed in an amount of 100 µL/well into the plate, followed by reacting at room temperature for 1 hour. Thereafter, the plate was washed with PBST Buffer five times, and TMB Soluble Reagent (High Sensitivity) (Scy Tek) was dispensed in an amount of 100 µL/well into the plate, and was then reacted at room temperature in a dark place for 3 minutes. Thereafter, TMB Stop Buffer (Scy Tek) was added in an amount of 100 µL/well into the plate, and was then shaken using a shaker for 1 minute. Subsequently, the absorbance at 450 nm (ref.: 620 nm) was measured using a plate reader.

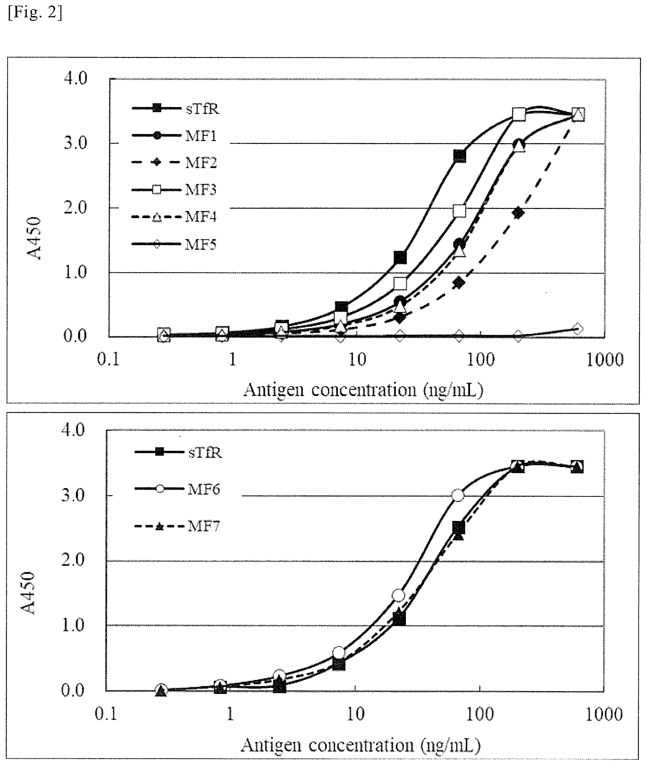

As a result, as shown in Fig. 2, a reduction in the reactivity of the TfR436 antibody with the TfR mutant fragment MF5 was observed, but such a reduction in the reactivity of the TfR436 antibody was not observed with other mutant fragments. That is to say, if the amino acids at positions 629, 630 and 633 of TfR are substituted with other amino acids, the TfR436 antibody cannot recognize it as TfR. It was suggested that the amino acids at positions 629 to 633 are an epitope which is recognized by the TfR436 antibody.

### Example 2: Comparison between TfR436 antibody and the comparative antibody in terms of inhibition of Tf-TfR binding

### (1) Production of comparative antibody A24

The Patent Document US 2008/0193453 discloses an A24 antibody reacting against human TfR. In order to compare the TfR436 antibody with the A24 antibody, the deposited hybridomas were obtained, and the A24 antibody was produced. Specifically, hybridomas were seeded in an RPMI1640 (GIBCO) medium supplemented with 10% FBS, to a cell concentration of 1 to 2 x 10⁵/mL, and were then cultured at 37°C in a 5%CO₂ incubator. After completion of cell expansion, the cells were recovered by centrifugation, and were then washed with PBS twice. Thereafter, the resulting cells were further subjected to cell expansion in the serum-free medium COSMEDIUM 005 (Cosmo Bio) supplemented with 0.5% Nutridoma-CS (Roche) to result in a volume of 550 mL. Five days after the cells became confluent, a culture supernatant was recovered by centrifugation.

The recovered supernatant was applied onto a protein A carrier (Ab-Capcher ExTra; ProteNova), and an antibody which bound to protein A was eluted with a 0.1 M glycine-HCl buffer (pH 2.7), and was promptly neutralized with a 1 M Tris-HCl buffer (pH 8.5). Thereafter, using Ultracel ultrafiltration disk (Merck Millipore), the buffer was exchanged with PBS.

### (2) Comparison between TfR436 antibody and the antibody developed by another company in terms of inhibition of Tf-TfR binding

The sTfR described in Example 1 was adjusted with PBST to a concentration of 5.0 µg/mL, and the diluted solution was then dispensed in an amount of 100 µL/well into a MaxiSorp 96-well plate (Nunc). The plate was left at rest at 4°C overnight for immobilization. On the following day, the solid phase liquid was discarded, and using 200 µL/well 100% Block Ace (DS Pharma Biomedical), the resultant was left at rest at room temperature for blocking. One hour later, the resulting plate was washed with PBST Buffer five times, and IIRr-labeled Tf (2 µg/mL) was then dispensed in an amount of 50 µL/well into the plate. Further, the TfR436 antibody, the A24 antibody (two steps of dilution series from 10 µg/mL), or holo-Tf (Sigma; two steps of dilution series from 300 µg/mL) was added in an amount of 50 µL/well into the plate. The reaction was carried out at room temperature for 1 hour, and the plate was then washed with PBST Buffer five times. Thereafter, TMB Soluble Reagent (high sensitivity) was dispensed in an amount of 100 µL/well into the plate, and it was then reacted at room temperature in a dark place. Twenty-five minutes later, TMB Stop Buffer was added in an amount of 100 µL/well into the plate, and the plate was then shaken using a shaker for 1 minute. Subsequently, the absorbance at 450 nm (ref.: 620 nm) was measured using a plate reader.

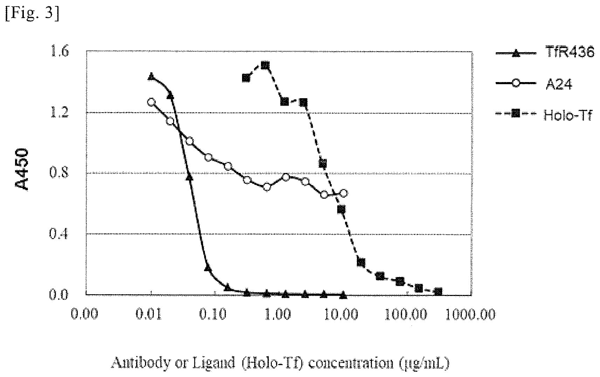

As a result, as shown in Fig. 3, the TfR436 antibody completely inhibited the binding of Tf-TfR at an extremely low dose (100 ng/mL). On the other hand, the A24 antibody could not completely inhibit the Tf-TfR binding, even though it was used at a dose of 10 µg/mL, and could inhibit only 50% of the Tf-TfR binding. Thus, it was suggested that the TfR436 antibody is excellent in terms of inhibition of the Tf-TfR binding.

### Example 3: Effects of ferric ammonium citrate on cell growth suppressing effect of TfR436 antibody

As shown in the above-described experimental results, the TfR436 antibody recognizes the amino acids at positions 629 to 633 of TfR and completely inhibits the binding of Tf-TfR. As a result of this inhibition, iron uptake into cells is completely inhibited. That is, the TfR436 antibody is an inhibitor of iron uptake into cells. Iron is a substance which is essential for the survival or growth of cells. If cells become deficient in iron, termination of the cell growth, or the cell death occurs. Whether the TfR436 antibody suppresses cell growth, or whether such cell growth suppression is caused by iron deficiency was examined by using ferric ammonium citrate which has been known as an iron donor in non-transferrin bound iron uptake. Specifically, K562 cells were suspended in a culture medium (RPMI1640, 10% FBS, and 1% P/S) such that the number of cells became 5000 cells/ml, and the obtained suspension was then seeded in an amount of 100 µl/well into a 96-well plate. A solution was prepared by diluting the TfR436 antibody by 5-fold serial dilution from 100 µg/ml, and 50 µL each of the diluted solution was added to the K562 cells (final concentration: 25 to 0.3 µg/ml). Thereafter, 50 µL of 120 µM ferric ammonium citrate (Wako Pure Chemical Industries, Ltd.) was further added thereto (final concentration: 30 µM). The obtained cells were cultured at 37°C in 5% CO₂ incubator for 96 hours, and then, individual wells were fully stirred. Thereafter, 150 µl of the cell culture solution was transferred into a V-bottom 96-well plate (Corning), and an aliquot (25 µl) of the cell culture solution was then sucked using FACS Calibur (BD). Thereafter, the number of events was measured. Four times the obtained number of events was defined to be the number of cells. The mean value of the number of cells in a well, to which neither the antibody nor ferric ammonium citrate was added, was set at 100%, and the cell growth rate in each treatment was then calculated.

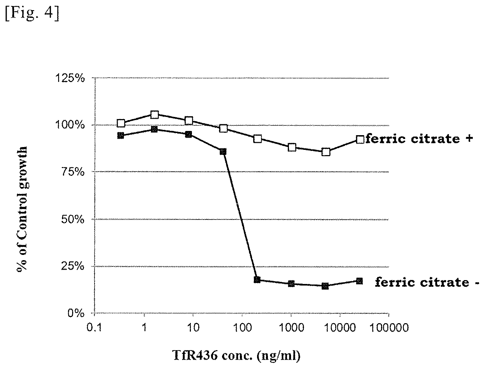

As a result, as shown in Fig. 4, the TfR436 antibody suppressed the growth of the K562 cells in a concentration-dependent manner. This termination of cell growth was released by addition of ferric ammonium citrate. From these results, it was suggested that the TfR436 antibody inhibits iron uptake into cells.

### Example 4: Change in iron amount in cell line by addition of TfR436 antibody

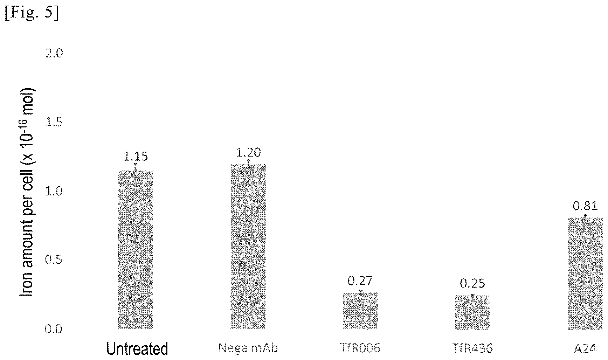

Using the K562 cell line, a change in the amount of ion in the cells by addition of the TfR436 antibody was examined. Specifically, the TfR436 antibody was added to a T150 flask (IMDM + 10%-FBS; 60 mL), in which K562 cells had been seeded to an amount of 0.5 x 10⁵ cells/mL, so that the final concentration of the TfR436 antibody became 5 µg/mL. As controls, a TfR006 antibody (described in Japanese Patent No. 5980202), an A24 antibody, a human monoclonal IgG1 antibody (Nega mAb), and a DPBS buffer (untreated) were prepared, and these controls were also added to the flask as in the case of the TfR436 antibody. The cells were cultured at 37°C in a 5% CO₂ incubator for 96 hours. Thereafter, the number of cells in each sample was counted, and 1.0 x 10⁷ cells were recovered. The recovered cells were washed with DPBS three times, and thereafter, 250 µL of Lysis M Reagent (Roche; cat.# 04 719 956 001) and 2.5 µL of 6 N HCl were added to and mixed with the cell pellets, followed by leaving the thus obtained mixture at rest at room temperature for 1 hour. After completion of centrifugation, the recovered supernatant was used in iron quantification. Iron quantification was carried out according to metalloassay iron measurement LS (ferrozine method) (Metallogenics; cat. #FE31M). The results are shown in Fig. 5.

As a result, as shown in Fig. 5, the amounts of iron in the K562 cells, into which the untreated control, Nega mAb, TfR006, TfR436, and A24 had been each added, were 1.15, 1.20, 0.27, 0.25, and 0.81 nmol, respectively. From these results, it was suggested that uptake of transferrin iron into the cells is suppressed by addition of the anti-TfR antibody, and that the amount of iron in the cells is reduced. In addition, it became clear that the TfR006 antibody and the TfR436 antibody have a higher effect of suppressing iron uptake than the A24 antibody. Moreover, these experimental results demonstrate that the TfR436 antibody has a higher effect of suppressing iron uptake than the TfR006 antibody.

## Claims

1. An agent for inhibiting iron uptake into cells, which comprises an antibody which recognizes the amino acids at positions 629 to 633 of a human transferrin receptor.

2. The agent for inhibition according to claim 1, which inhibits the binding between a human transferrin and a human transferrin receptor, so as to inhibit iron uptake into cells.

3. The agent for inhibition according to claim 1 or 2, wherein the antibody has a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.

4. The agent for inhibition according to any one of claims 1 to 3, wherein the antibody has a heavy chain as set forth in SEQ ID NO: 7 and a light chain as set forth in SEQ ID NO: 8.

5. The agent for inhibition according to any one of claims 1 to 4, wherein the antibody is a human antibody or a humanized antibody.

6. The agent for inhibition according to any one of claims 1 to 5, wherein the antibody is an antibody fragment selected from the group consisting of Fab, Fab', F(ab')₂, a single-chain antibody (scFv), a dimerized V region (Diabody), a disulfide stabilized V region (dsFv), and a peptide comprising CDR.

7. The agent for inhibition according to any one of claims 1 to 6, which is used for the treatment of a disease or a symptom associated with excessive iron uptake into cells.

8. An agent for inhibition the binding between a human transferrin and a human transferrin receptor, which comprises an antibody which recognizes the amino acids at positions 629 to 633 of a human transferrin receptor.

9. The agent for inhibition according to claim 8, wherein the antibody has a heavy chain first complementarity determining region (VH CDR1), a heavy chain second complementarity determining region (VH CDR2), and a heavy chain third complementarity determining region (VH CDR3), which are as set forth in SEQ ID NOs: 1, 2, and 3, respectively, and a light chain first complementarity determining region (VL CDR1), a light chain second complementarity determining region (VL CDR2), and a light chain third complementarity determining region (VL CDR3), which are as set forth in SEQ ID NOs: 4, 5, and 6, respectively.

10. The agent for inhibition according to claim 8 or 9, wherein the antibody has a heavy chain as set forth in SEQ ID NO: 7 and a light chain as set forth in SEQ ID NO: 8.

11. The agent for inhibition according to any one of claims 8 to 10, wherein the antibody is a human antibody or a humanized antibody.

12. The agent for inhibition according to any one of claims 8 to 11, wherein the antibody is an antibody fragment selected from the group consisting of Fab, Fab', F(ab')₂, a single-chain antibody (scFv), a dimerized V region (Diabody), a disulfide stabilized V region (dsFv), and a peptide comprising CDR.

13. The agent for inhibition according to any one of claims 8 to 12, which is used for the treatment of a disease or a symptom associated with excessive iron uptake into cells.
